# EUROPEAN PATENT APPLICATION

(11) **EP 4 279 497 A1**
(43) Date of publication of application: **22.11.2023**
(21) Application number: 22739740.3
(22) Date of filing: 14.01.2022
(51) Int. Cl.: C07K 7/08, C07K 19/00, C07K 16/00, G01N 33/68, G01N 33/58, G01N 33/53

(54) **SWITCHING PEPTIDE AND MULTIPLEX IMMUNOASSAY METHOD USING SAME**

(30) Priority: 14.01.2021 KR 20210005036
(71) Applicant: Optolane Technologies Inc., Bundang-gu Seongnam-si, Gyeonggi-do 13494 (KR)
(72) Inventor: PYUN, Jae-Chul, Seoul 06521 (KR)
(74) Representative: Schiweck Weinzierl Koch Patentanwälte Partnerschaft mbB
(86) International application number: PCT/KR2022/000692
(87) International publication number: WO 2022/154547

(57) **Abstract**

Disclosed is a switching peptide applicable to multiplex immunoassay method. The switching peptide comprises: a peptide compound capable of reversibly binding to a binding antibody; and a mass label substance bound to the peptide compound.

## Description

### [Technical Field]

The present disclosure relates to a switching peptide that may be used to analyze a target antigen substance using an immune response and a multiplex immunoassay method using the same.

### [Background Art]

Immunoassay has been used for bio-testing such as various immunodiagnostic tests or environmental monitoring in the field of biotechnology. The immunoassay has the advantage of being able to quantitatively analyze a specific target substance by using the specificity of an antigen-antibody reaction and having higher sensitivity than other analysis methods.

The immunoassay may be used for various diagnoses such as diagnosing cancer markers, infectious diseases, thyroid function, anemia, allergy, pregnancy, drug abuse, and gout. In order to use the immunoassay for diagnosis or analysis of various types of antigens, it is necessary to prepare antibodies having specific reactivity to each of the antigens.

However, such a method has limitations in detecting modernized diseases that have rapidly been diversified recently, or viruses having multiple mutants. In particular, in the case of the virus, it is often impossible to find an antibody having a specific binding property with the virus.

In addition, when the antigen-antibody reaction is used, an additional step of binding the antibody to a label may be required. If the binding property between the label and the antibody is poor, the accuracy or reliability of immunoassay analysis may be deteriorated.

Due to the aforementioned problems, there is also a problem of developing various types of labels capable of binding to various antibodies, respectively.

In addition, many studies have been conducted on multiplex immunoassay capable of simultaneously analyzing a plurality of analytes for rapid medical diagnosis and environmental monitoring. As conventional multiplex immunoassay, a method of binding an antibody to a solid support such as a bead has been mainly used. In this case, the amount of fluorescence was adjusted according to the type of assay, and each antibody was bound to different beads to identify the reaction, and sandwich-type fluorescence immunoassay was performed on the beads to quantify the amount of bound antigen. In addition, in general, a sequential analysis method using a flow cytometry has been used as a method of measuring the amount of fluorescence of a bead and the amount of fluorescence generated on the bead by immunoassay. However, such a conventional multiplex immunoassay had a problem in that it takes a long time to analyze a plurality of analytes.

### [Disclosure]

### [Technical Problem]

One object of the present disclosure is to provide a plurality of switching peptides comprising peptide compounds capable of selectively and reversibly binding to a Fab region of a binding antibody and mass labels having different molecular weights.

Another object of the present disclosure is to provide a multiplex immunoassay method capable of simultaneous analysis of a plurality of antigens using the switching peptides.

### [Technical Solution]

A switching peptide according to an embodiment of the present disclosure comprises: a peptide compound capable of reversibly binding to a binding antibody; and a mass label bound to the peptide compound.

In one embodiment, the peptide compound may be selectively and reversibly bound to a fragment antigen-binding (Fab) region of the binding antibody.

In one embodiment, the peptide compound may have an amino acid sequence capable of specifically and reversibly binding to one or more of first to fourth framework regions (FR1, FR2, FR3, and FR4) of a light chain or heavy chain of the binding antibody.

In one embodiment, the peptide compound may comprise one or more selected from the group consisting of a first peptide compound having a first amino acid sequence having homology to an amino acid sequence of the second light chain variable framework region (VL-FR2); a second peptide compound having a second amino acid sequence having homology to an amino acid sequence of the third or fourth light chain variable framework region (VL-FR3, VL-FR4) ; a third peptide compound having a third amino acid sequence having homology to an amino acid sequence of the second heavy chain variable framework region (VH-FR2); and a fourth peptide compound having a fourth amino acid sequence having homology to an amino acid sequence of the third or fourth heavy chain variable framework region (VH-FR3, VH-FR4).

In one embodiment, the peptide compound may comprise 14 to 20 amino acids.

In one embodiment, the peptide compound may have a molecular weight of 1,650 to 2,500 Da.

In one embodiment, the mass label may comprise an amino acid compound.

A multiplex immunoassay method according to an embodiment of the present disclosure includes: a first step of immobilizing N different binding antibodies to which N different switching peptides are respectively bound, on a substrate;
a second step of treating the N binding antibodies with a detection sample solution containing one or more target antigens; and
a third step of performing quantitative analysis on the target antigens contained in the detection sample solution by identifying and quantitatively analyzing switching peptides released from the binding antibodies by mass spectrometry due to binding of the target antigens, wherein each of the switching peptides may comprise a peptide compound that reversibly bound to the binding antibodies and a mass label bound to the peptide compound, and the mass labels of the switching peptides may each comprise amino acid compounds having different molecular weights.

In one embodiment, the binding antibodies comprise first and second binding antibodies that are different from each other, and the switching peptides comprise a first switching peptide comprising a first peptide compound that reversibly bound to the Fab region of the first binding antibody and a first mass label bound thereto and having a first molecular weight, and a second switching peptide comprising a second peptide compound that reversibly bound to the Fab region of the second binding antibody and a second mass label bound thereto and having a second molecular weight different from the first molecular weight, wherein the first peptide compound and the second peptide compound may be identical to each other.

In one embodiment, in the second step, the detection sample solution containing first and second target antigens that specifically react with the first and second binding antibodies is coated on the first and second binding antibodies to specifically react the first and second target antigens with the first and second binding antibodies, respectively, and during the second step, the first switching peptide may be quantitatively released from the first binding antibody depending on an amount of the first target antigen, and the second switching peptide may be quantitatively released from the second binding antibody depending on an amount of the second target antigen.

In one embodiment, during the third step, quantitative analysis of each of the first and second target antigens contained in the detection sample solution may be performed by identifying and quantitatively analyzing the first and second switching peptides released from the first and second binding antibodies, respectively, by mass spectrometry.

In one embodiment, mass spectrometry of the first and second switching peptides may be performed by a method of laser desorption/ionization mass spectrometry or liquid chromatography mass spectrometry.

In one embodiment, mass spectrometry of the first and second switching peptides may be performed by a method of laser desorption/ionization mass spectrometry using an inorganic matrix, wherein the inorganic matrix may comprise semiconductor nanowires and porous metal nanoparticles bound to a surface of the semiconductor nanowires.

### [Advantageous Effects]

According to a switching peptide of the present disclosure and an immunoassay method using the same, when different antigens react specifically to the corresponding binding antibodies, respectively, switching peptides each modified with mass labels having different molecular weights may be quantitatively released from the binding antibodies, and the released switching peptides may be identified and quantitatively analyzed by mass spectrometry to simultaneously analyze a plurality of antigens, thereby significantly reducing the analysis time for the antigens.

### [Description of Drawings]

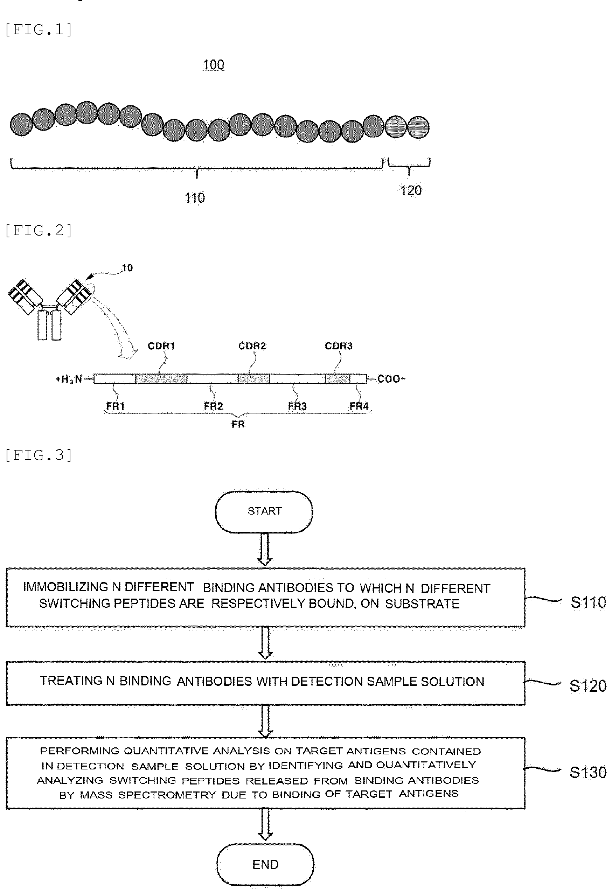

FIG. 1 is a diagram for explaining a switching peptide according to an embodiment of the present disclosure.
FIG. 2 is a diagram for explaining a binding antibody.

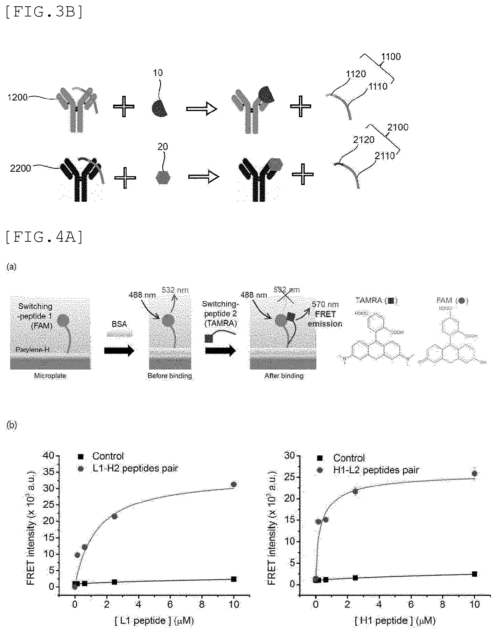

FIGS. 3A and 3B are a flowchart and a schematic diagram for explaining an immunoassay method according to an embodiment of the present disclosure, respectively, and FIG. 3C is a diagram for explaining one embodiment of a matrix applied to laser desorption/ionization mass spectrometry for switching peptides.

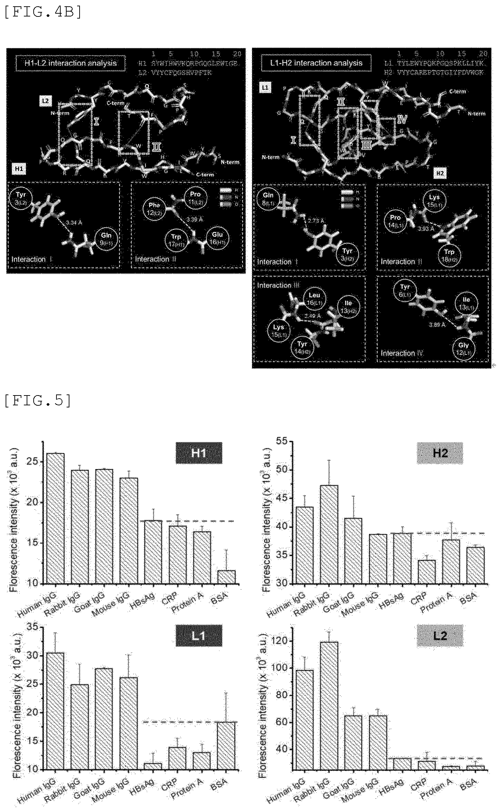

FIG. 4A is diagrams illustrating a method for confirming a specific interaction between L1 and H2 peptides and a specific interaction between L2 and H1 peptides and the measurement results thereof, and FIG. 4B is diagrams for explaining a specific interaction between L1 and H2 peptides and a specific interaction between L2 and H1 peptides analyzed using PyMOL software.
FIG. 5 is graphs illustrating a fluorescence intensity measured at a bottom of a microplate after reacting lgGs from human, rabbit, goat, and mouse, which are isolated and immobilized on the microplate, respectively, and other proteins (human hepatitis B antigen, C-reactive protein, protein-A, BSA) with fluorescently labeled four types of peptide compounds (H1, H2, L1, L2) synthesized in Example 1.

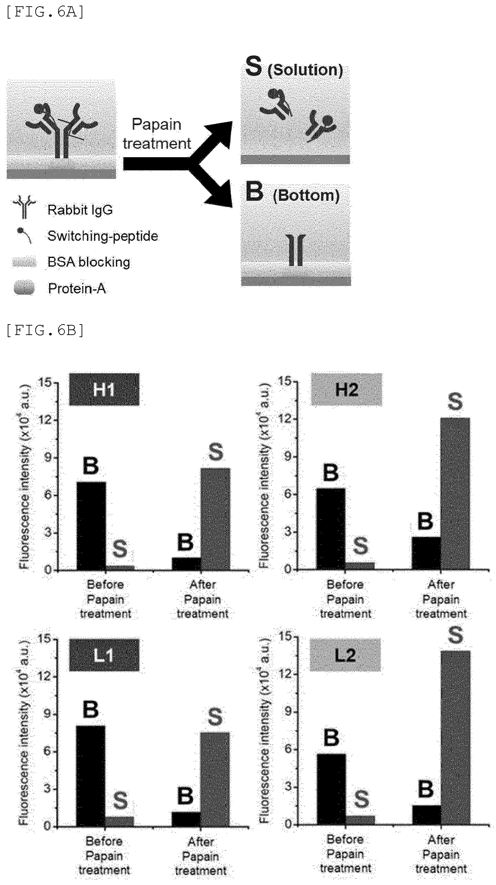

FIG. 6A is a schematic diagram illustrating states before and after treatment of the antibody to which the four types of peptides of Example 1 were bound, respectively, with papain, which is a protease, and FIG. 6B is graphs illustrating a fluorescence intensity measured at a bottom (B) of the microplate and in a solution (S) after the papain treatment.

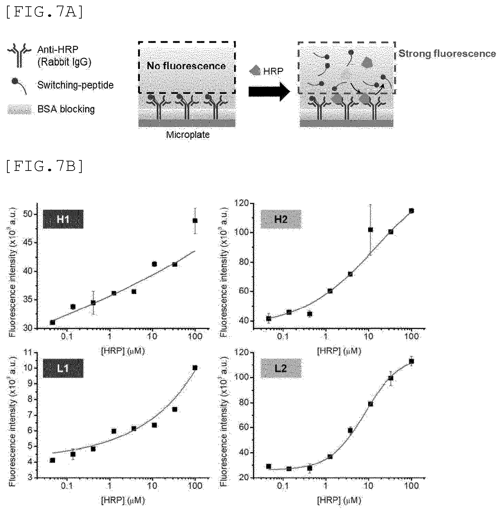

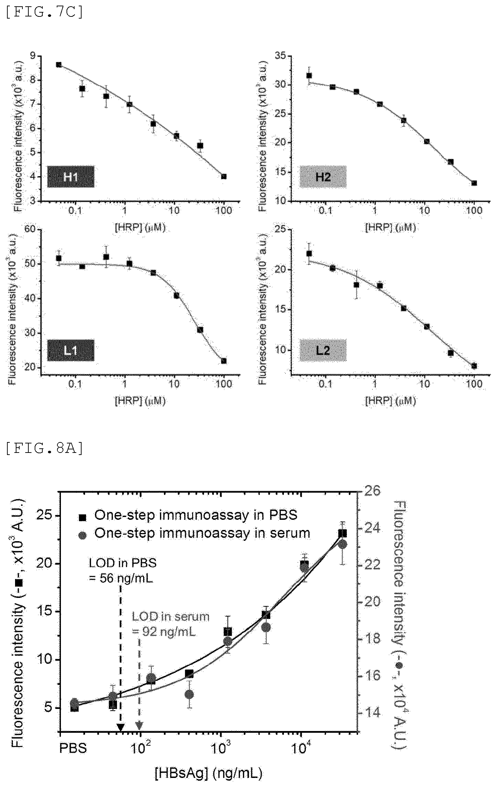

FIG. 7A is a schematic diagram for explaining an immunoassay method using fluorescently labeled peptide compounds of Example 1, and FIGS. 7B and 7C are graphs illustrating the fluorescence intensities in a solution and at a bottom of the microplate depending on a concentration of the antigen measured by the immunoassay method illustrated in FIG. 7A.
FIGS. 8A to 8D are graphs illustrating experimental results for hepatitis B surface antigen (HBsAg) detected using an immunoassay using the peptide compounds of Example 1.

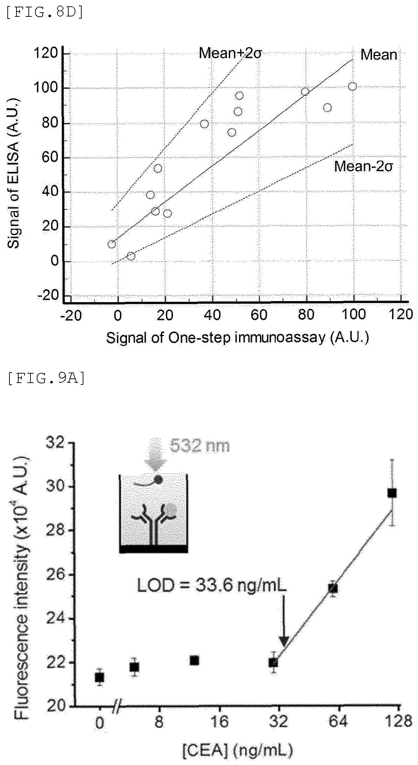

FIGS. 9A to 9C are graphs illustrating a limit of detection (LOD) and an immunoassay baseline in one-step immunoassay using the switching peptide according to the present disclosure.

### [Best Mode for Invention]

Hereinafter, exemplary embodiments of the present disclosure will be described in detail with reference to the accompanying drawings. The present disclosure may be variously modified and have several exemplary embodiments. Therefore, specific exemplary embodiments of the present disclosure will be illustrated in the accompanying drawings and be described in detail in the specification. However, it is to be understood that the present disclosure is not limited to a specific disclosed form, but includes all modifications, equivalents, and substitutions without departing from the scope and sprit of the present disclosure. Similar reference numerals have been used for similar components in describing each drawing. In the accompanying drawings, dimensions of the structures have been enlarged as compared with the actual dimensions for clarity of the present disclosure.

Terms as used herein are used only in order to describe specific exemplary embodiments rather than limiting the present disclosure. Singular forms include plural forms unless the context clearly indicates otherwise. It should be understood that term "include" or "has" as used herein specify the presence of features, numerals, steps, operations, components described herein, or combinations thereof, but does not preclude the presence or addition of one or more other features, numerals, steps, operations, components, or combinations thereof.

Unless being defined otherwise, it is to be understood that all the terms as used herein including technical and scientific terms have the same meanings as those that are generally understood by one of ordinary skill in the art to which the present disclosure pertains. Terms generally used and defined by a dictionary should be interpreted as having the same meanings as meanings within a context of the related art and should not be interpreted as having ideal or excessively formal meanings unless being clearly defined otherwise in the present specification.

### <Switching peptide>

FIG. 1 is a diagram for explaining a switching peptide according to an embodiment of the present disclosure, and FIG. 2 is a diagram for explaining a binding antibody.

Referring to FIGS. 1 and 2, a switching peptide 100 according to an embodiment of the present disclosure may comprise a peptide compound 110 capable of reversibly binding to a binding antibody 10, and a mass label 120 bound to the peptide compound 110. The switching peptide 100 may be reversibly bound to the binding antibody 10 that specifically bound to a target antigen, and thus may be quantitatively released from the binding antibody 10 when the target antigen is specifically bound to the binding antibody. As a result, the electrochemical characteristics of a test sample including the target antigen may be changed by a redox reaction mediated by the fluorescent label 120 of the switching peptide 100 released from the binding antibody 10.

The binding antibody 10 is a compound capable of inducing an immunological effector mechanism by binding to or reacting with a specific antigenic determinant such as an epitope of the target antigen. The binding antibody may be monospecific or polyspecific.

In one embodiment, the binding antibody 10 may comprise an antibody, an antibody derivative, or a fragment thereof. The binding antibody 10 may be an intact immunoglobulin molecule or, alternatively, may comprise components of intact antibodies such as Fab, Fab', F(ab')₂, Fc, F (v), N-glycan structure, paratope, etc., or at least some of the components.

In one embodiment, the binding antibody may be a human antibody, a non-human antibody, or a humanized non-human antibody.

The human antibody may comprise antibodies produced by humans, or synthetic antibodies having amino acid sequences synthesized using any technique. This definition of the human antibody specifically excludes humanized antibodies comprising non-human antigen-binding residues. The human antibody may be produced using a variety of techniques known in the art, including phage-display libraries. The non-human antibody may be an antibody obtained from sources of various species, for example, rodents, rabbits, cattle, sheep, pigs, dogs, non-human mammals, or birds. A "humanized" form of the non-human antibody may be a chimeric antibody that contains minimal sequence derived from non-human immunoglobulin. In one embodiment, the humanized antibody may be a human immunoglobulin (recipient antibody) in which residues from a hypervariable region of the recipient are replaced with the hypervariable region of the aforementioned non-human antibody (donor antibody) having a desired specificity, affinity and/or capacity. In some cases, framework residues of a human immunoglobulin may be replaced by corresponding non-human residues. In addition, the humanized antibody may comprise residues not found in either the recipient antibody or the donor antibody. These modifications may be made to further refine antibody performance, such as binding affinity.

In one embodiment, the binding antibody 10 may be an antibody molecule, an immunoglobulin molecule, a fragment thereof, or an aggregate of one or more of them. The binding antibody 10 may have a unique structure that enables specific binding to the target antigen, and each binding antibody 10 may comprise two light chains of identical structure and two heavy chains of identical structure. The heavy chain and the light chain may comprise a variable region and a constant region, respectively. The variable regions of the heavy chain and the light chain may be combined to form an antigen binding site. In the binding antibody 10, a portion to which a target antigen is bound by a protease such as papain may be separated into an antibody binding fragment (Fab) region and a fragment crystallizable (Fc) region, which is a crystallized supporting pillar portion, where the antigen binding site may be included in the antibody binding fragment (Fab) region of the antibody.

Meanwhile, each of the variable regions of the heavy and light chains may have a structure in which three complementarity-determining regions (CDRs) that are hypervariable regions (HVRs), and four framework regions (FRs) structurally supporting the complementarity-determining regions are alternately arranged, respectively. The complementarity-determining regions have different amino acid sequences for each antibody, and thus may be specifically bound to each antigen, whereas the framework regions have conserved amino acid sequences according to subfamilies of organisms, so antibodies of organisms belonging to the same subfamilies have the same or similar amino acid sequences.

For the convenience of description below, in the variable region of the light chain of the binding antibody 10, the four framework regions arranged sequentially apart from the N-terminus, are referred to as first to fourth light chain variable framework regions (VL-FR1, VL-FR2, VL-FR3, and VL-FR4), respectively, and the three complementarity determining regions, which are located between the first to fourth light chain variable framework regions (VL-FR1, VL-FR2, VL-FR3, and VL-FR4), respectively, and sequentially disposed from the N-terminus, are referred to as first to third light chain variable complementarity determining regions (VL-CDR1, VL-CDR2, VL-CDR3), respectively. In addition, in the variable region of the heavy chain of the binding antibody 10, the four framework regions arranged sequentially apart from the N-terminus are referred to as first to fourth heavy chain variable framework regions (VH-FR1, VH-FR2, VH-FR3, and VH-FR4), respectively, and the three complementarity-determining regions, which are located between first to fourth heavy chain variable framework regions (VH-FR1, VH-FR2, VH-FR3, and VH-FR4), respectively, and sequentially disposed from the N-terminus, are referred to as first to third heavy chain variable complementarity-determining regions (VH-CDR1, VH-CDR2, VH-CDR3), respectively.

The peptide compound 110 may have an amino acid sequence capable of selectively and reversibly binding to the fragment antigen-binding (Fab) region of the binding antibody 10.

In one embodiment, the peptide compound 110 may have an amino acid sequence capable of specifically and reversibly binding to one or more of first to fourth framework regions (FR1, FR2, FR3, and FR4) of a light chain or heavy chain of the binding antibody 10. For example, the peptide compound 110 may comprise an amino acid sequence having homology to one or more of the first to fourth framework regions (FR1, FR2, FR3 and FR4) of the light chain or heavy chain of the binding antibody 10. In this case, the peptide compound 110 may be reversibly bound to one or more of the first to fourth framework regions (FR1, FR2, FR3, and FR4) of the light chain or heavy chain. Accordingly, when the target antigen is bound to the binding antibody 10, the peptide compound 10 bound to the binding antibody 10 may be quantitatively released from the binding antibody 10 depending on the amount of the antigen bound to the binding antibody 10.

In one embodiment, the peptide compound 110 may comprise one or more selected from a first peptide compound L1 having a first amino acid sequence having homology to an amino acid sequence of the second light chain variable framework region (VL-FR2), a second peptide compound L2 having a second amino acid sequence having homology to an amino acid sequence of the third or fourth light chain variable framework region (VL-FR3, VL-FR4), a third peptide compound H1 having a third amino acid sequence having homology to an amino acid sequence of the second heavy chain variable framework region (VH-FR2), and a fourth peptide compound H2 having a fourth amino acid sequence having homology to an amino acid sequence of the third or fourth heavy chain variable framework region (VH-FR3, VH-FR4).

In the binding antibody 10, the second light chain variable framework region (VL-FR2) and the third or fourth heavy chain variable framework region (VH-FR3, VH-FR4) are located adjacent to each other and interact with each other, and the third or fourth light chain variable framework region (VL-FR3, VL-FR4) and the second heavy chain variable framework region (VH-FR2) are located adjacent to each other and interact with each other. Thus, the first peptide compound L1 having a first amino acid sequence having homology to the second light chain variable framework region (VL-FR2) of the binding antibody 10 may selectively interact with the third or fourth heavy chain variable framework region (VH-FR3, VH-FR4) of the binding antibody 10, the second peptide compound L2 having a second amino acid sequence having homology to the third or fourth light chain variable framework region (VL-FR3, VL-FR4) of the binding antibody 10 may selectively interact with the second heavy chain variable framework region (VH-FR2) of the binding antibody 10, the third peptide compound H1 having a third amino acid sequence having homology to the second heavy chain variable framework region (VH-FR2) of the binding antibody 10 may selectively interact with the third or fourth light chain variable framework region (VL-FR3, VL-FR4) of the binding antibody 10, and the fourth peptide compound H2 having a fourth amino acid sequence having homology to the third or fourth heavy chain variable framework region (VH-FR3, VH-FR4) of the binding antibody 10 may selectively interact with the second light chain variable framework region (VL-FR2) of the binding antibody 10.

In one embodiment, when the binding antibody 10 is derived from an organism belonging to subfamilies of animals including humans, the peptide compound 110 may comprise about 10 to 25 amino acids and may have a molecular weight of about 1,600 to 3,000 Da. For example, the peptide compound 110 may comprise about 14 to 20 amino acids and may have a molecular weight of about 1,650 to 2,500 Da. In one embodiment, the first to fourth peptide compounds (L1, L2, H1, H2) may comprise amino acid sequences as shown in Table 1 below.

**[Table 1]**

| Switching peptide | Amino acid sequence | Molecular weight | Gibbs free energy (Kcal/mol) |
|---|---|---|---|
| H1 | SYWIH WVKQR PGQGL EWIGE | 2469.7 | △ G=-17.91 |
| H2 | VYYCA REPTG TGIYF DVWGK | 2325.6 | △ G=-17.19 |
| L1 | TYLEW YPQKP GQSPK LLIYK | 2452.8 | △ G=-15.37 |
| L2 | VYYCF QGSHV PFTK | 1675.9 | △ G=-13. 05 |

Meanwhile, since the framework regions (FR1, FR2, FR3, FR4) of the light and heavy chains of the antibodies have conserved amino acid sequences according to subfamilies of organisms, the peptide compound 110 may be commonly applied to antibodies derived from different species of organisms belonging to the same subfamilies. Therefore, even when the peptide compound 110 is synthesized from antibodies of goat, mouse, rabbit, etc., it can exhibit the same actions and effects as described above for antibodies of human. The mass label 120 may be bound to an end of the peptide compound 110. In one embodiment, the mass label 120 may comprise one or more amino acid compounds. In this case, when the peptide compound 110 is synthesized, the mass label 120 may be modified at an end of the peptide compound 110.

### <Multiplex immunoassay method>

FIGS. 3A and 3B are a flowchart and a schematic diagram for explaining an immunoassay method according to an embodiment of the present disclosure, respectively, and FIG. 3C is a diagram for explaining one embodiment of a matrix applied to laser desorption/ionization mass spectrometry for switching peptides.

Referring to FIGS. 3A and 3B, the immunoassay method according to an embodiment of the present disclosure includes: a first step (S110) immobilizing N different binding antibodies to which N different switching peptides are respectively bound, on a substrate; a second step (S120) of treating the N binding antibodies with a detection sample solution; and a third step (S130) of performing quantitative analysis on the target antigens contained in the detection sample solution by identifying and quantitatively analyzing switching peptides released from the binding antibodies by mass spectrometry due to binding of the target antigens. N may be a natural number equal to or greater than 2. Hereinafter, for the convenience of description, a case in which N is 2 will be described as an example, as illustrated in FIG. 3B.

In the first step (S110), a first binding antibody 1200 to which the first switching peptide 1100 is bound and a second binding antibody 2200 to which a second switching peptide 2100 is bound, may be immobilized on the substrate.

The first switching peptide 1100 may comprise the first peptide compound 1110 and a first mass label 1120 bound thereto, and the second switching peptide 2100 may comprise a second peptide compound 2110 and a second mass label 2120 bound thereto. Each of the first and second switching peptides 1100 and 2100 may have substantially the same structure as the switching peptide 100 described with reference to FIG. 1. Therefore, an overlapped description thereof will be omitted below.

In one embodiment, the first peptide compound 1110 and the second peptide compound 2110 may be the same, and the first mass label 1120 and the second mass label 2120 may be different from each other. For example, the first mass label 1120 may be a first amino acid compound having a first molecular weight, and the second mass label 2120 may be a second amino acid compound having a second molecular weight different from the first molecular weight. Meanwhile, the first and second amino acid compounds may be composed of a single amino acid or two or more amino acids.

The first peptide compound 1110 of the first switching peptide 1100 may have an amino acid sequence capable of selectively and reversibly binding to a fragment antigen-binding (Fab) region of the first binding antibody 1200. Accordingly, when a first target antigen 10 that specifically reacts with the first binding antibody 1200 is bound to the first binding antibody 1200, the first switching peptide 1100 may be quantitatively released from the first binding antibody 1200 depending on an amount of the first target antigen 10 bound to the first binding antibody 1200.

The second peptide compound 2100 of the second switching peptide 2110 may have an amino acid sequence capable of selectively and reversibly binding to a fragment antigen-binding (Fab) region of the second binding antibody 2200. Accordingly, when a second target antigen 20 that specifically reacts with the second binding antibody 2200 is bound to the second binding antibody 2200, the second switching peptide 2100 may be quantitatively released from the second binding antibody 2200 depending on an amount of the second target antigen 20 bound to the second binding antibody 2200.

A method of immobilizing the first and second binding antibodies 1200 and 2200 on the substrate is not particularly limited. For example, each of the first and second binding antibodies 1200 and 2200 may be directly bound to the substrate or may be bound to the substrate through a linker compound.

In the second step (S120), a detection sample solution containing the first and second target antigens 10 and 20 may be coated on the first and second binding antibodies 1200 and 2200 to specifically react the first and second target antigens 10 and 20 with the first and second binding antibodies 1200 and 2200, respectively.

In this case, as described above, the first switching peptide 1100 may be quantitatively released from the first binding antibody 1200 depending on the amount of the first target antigen 10 bound to the first binding antibody 1200, and the second switching peptide 2100 may be quantitatively released from the second binding antibody 2200 depending on the amount of the second target antigen 20 bound to the second binding antibody 2200. That is, the first and second switching peptides 1100 and 1200 released from the first and second binding antibodies 1200 and 2200 may exist in the detection sample solution after the reaction, and the amounts of the first and second switching peptides 1100 and 1200 present in a free state in the detection sample solution may vary depending on the concentrations of the first and second antigens 10 and 20.

In the third step (S130), quantitative analysis may be performed on each of the first and second target antigens 10 and 20 contained in the detection sample solution by identifying and quantitatively analyzing the first and second switching peptides 1100 and 1200 in a free state present in the detection sample solution after the reaction, by mass spectrometry. Amass spectrometry method for the first and second switching peptides 1100 and 1200 is not particularly limited. For example, mass spectrometry of the first and second switching peptides 1100 and 1200 may be performed by methods such as laser desorption/ionization mass spectrometry (LDI-MS) and liquid chromatography mass spectrometry (LC-MS).

In one embodiment, mass spectrometry of the first and second switching peptides 1100 and 1200 may be performed through laser desorption/ionization mass spectrometry (LDI-MS) using an inorganic matrix. In this case, as illustrated in FIG. 3C, the inorganic matrix 50 may comprise semiconductor nanowires 51 and porous metal nanoparticles 52 bound to the surface of the semiconductor nanowires 51.

The semiconductor nanowires 51 may be formed of a semiconductor material having photocatalytic activity. For example, the semiconductor nanowires 51 may be formed of one or more semiconductor materials selected from titanium dioxide (TiO₂), zinc oxide (ZnO), zirconium dioxide (ZrO₂), tungsten trioxide (WO₃), etc.

In one embodiment, the semiconductor nanowires 51 may be formed of titanium dioxide. The titanium dioxide nanowire are a semiconductor having a bandgap energy of about 3.2 eV, and may absorb ultraviolet laser with a wavelength of about 337 nm, which is mainly used in laser desorption/ionization mass spectrometry (LDI-MS), and may also absorb laser energy due to its photocatalytic activity and promote photochemical reaction of the analyte, thereby promoting ionization of the analyte.

The porous metal nanoparticles 52 may be bound to the surface of the semiconductor nanowiress 51. The porous metal nanoparticles 52 may be formed of a metal material having low specific heat and high heat conductivity, and may have pores formed on the surface or inside. For example, the porous metal nanoparticles 52 may be formed of a metal material having excellent affinity for biomolecules, such as gold (Au), silver (Ag), titanium (Ti), rhodium (Rh), niobium (Nb), molybdenum (Mo), tantalum (Ta), zirconium (Zr), tungsten (W), tin (Sn), etc.

In one embodiment, the porous metal nanoparticles 52 may have a size equal to or smaller than a heat diffusion length thereof. In this case, it is possible to uniformly heat the porous metal nanoparticles 52 by laser irradiation, thereby reducing a threshold value of laser intensity required for detachment of the analyte. For example, the porous metal nanoparticles 52 may have a size of about 20 to 200 nm.

In one embodiment, the inorganic composite matrix 50 may comprise the semiconductor nanowires 51 and the porous metal nanoparticles 52 in a weight ratio of about 1:0.01 to 1:0.05. For example, the inorganic composite matrix 50 may comprise the semiconductor nanowires 51 and the porous metal nanoparticles 52 in a weight ratio of about 1:0.02 to 1:0.03, and preferably 1:0.0208 to 1:0.0211.

The inorganic matrix 50 may have improved local heat dissipation ability due to high photothermal conversion efficiency and low specific heat of the porous metal nanoparticles 52, and may improve separation ability of a charge carrier due to a Schottky potential barrier formed at an interface between the semiconductor nanowires 51 and the porous metal nanoparticles 52. As a result, the desorption and ionization ability of the analyte may be improved.

In addition, a phenomenon of surface restructuring and melting of the porous metal nanoparticles 52 induced by a laser caused by photothermal conversion of the porous metal nanoparticles 52, causes a change in strain in the porous metal nanoparticles 52, which may generate a change in strain and oxygen vacancies in the adjacent semiconductor nanowires 51, and oxygen vacancies in the semiconductor nanowires 51 thus generated may generate a shallow trap level for electrons. A trap level by the oxygen vacancies together with the Schottky barrier may further lower a recombination rate of light-induced charges.

In addition, dangling bonds present on the surface of the porous metal nanoparticles 52 may retard thermal electron movement, and as a result, a thermal confinement effect into the porous metal nanoparticles 52 may be enhanced. Therefore, the porous metal nanoparticles 52 may induce a higher surface temperature due to increased surface voidage during laser irradiation compared to non-porous metal nanoparticles.

According to the switching peptide of the present disclosure and the immunoassay method using the same, when different antigens react specifically to the corresponding binding antibodies, respectively, switching peptides each modified with mass labels having different molecular weights may be quantitatively released from the binding antibodies, and the released switching peptides may be identified and quantitatively analyzed by mass spectrometry to simultaneously analyze a plurality of antigens, thereby significantly reducing the analysis time for the antigens.

Hereinafter, specific embodiments of the present disclosure will be described in detail. However, the following examples are merely some embodiments of the present disclosure, and the scope of the present disclosure is not limited to the following examples.

### [Mode for Invention]

### [Example 1]

Four types of peptides (H1, H2, L1, L2) as shown in Table 1 were synthesized. Specifically, the H1 peptide was synthesized to have an amino acid sequence having homology to the FR2 region of the heavy chain, the H2 peptide was synthesized to have an amino acid sequence having homology to the FR3 or FR4 region of the heavy chain, the L1 peptide was synthesized to have an amino acid sequence having homology to the FR2 region of the light chain, and the L2 peptide was synthesized to have an amino acid sequence having homology to the FR3 or FR4 region of the light chain.

A change in Gibbs free energy (ΔG) was -17.91 kcal/mol for the H1 peptide compound, -17.19 kcal/mol for the H2 peptide, 15.37 kcal/mol for the L1 peptide, and -13.05 kcal/mol for the L2 peptide.

### [Experimental Example 1]

FIG. 4A is diagrams illustrating a method for confirming a specific interaction between L1 and H2 peptides and a specific interaction between L2 and H1 peptides and the measurement results thereof, and FIG. 4B is diagrams for explaining a specific interaction between L1 and H2 peptides and a specific interaction between L2 and H1 peptides analyzed using PyMOL software. Here, at the ends of the L1 and L2 peptides, a first fluorescent label, FAM (λₑₓ = 488 nm, λₑₘ = 532 nm) was modified, and at the ends of the H1 and H2 peptides, a second fluorescent label, TAMRA (λₑₓ = 532 nm, λₑₘ = 570 nm) was modified. Meanwhile, the first peptide (L1 or L2) was immobilized on a parylene-H-coated microplate through a covalent bond between a formyl group of parylene-H and a primary amine group of a lysine residue in the peptide, and the second peptide (H2 or H1) was incubated with the immobilized first peptide (L1 or L2) to allow specific interactions between them. On the other hand, it can be seen that a specific interaction between an immobilized first peptide (L1 or L2) and a second peptide (H2 or H1) incubated therewith may be determined by whether the FRET effect with green fluorescence emission from TAMRA (λₑₘ = 570 nm) modified to the second peptide was observed at the bottom of the microplate, and when a specific interaction between the first and second peptides occurs, the FRET effect was observed at the bottom of the microplate.

Referring to FIG. 4A, the interaction between the L1 and H2 peptides was observed by changing the concentration of the L1 peptide, and the FRET intensity increased with increasing the concentration of the L1 peptide. These results indicate that a specific interaction between the L1 and H2 peptides has occurred.

Also, the interaction between the H1 and L2 peptides was observed by changing the concentration of the H1 peptide, and the FRET intensity was observed to increase with increasing the concentration of the H1 peptide. These results also indicate that a specific interaction between the H1 and L2 peptides has occurred.

From the above results, it can be seen that the L1 peptide is selectively and reversibly bound to the FR3 or FR4 region of the heavy chain (corresponding to the H2 peptide), the L2 peptide is selectively and reversibly bound to the FR 2 region of the heavy chain (corresponding to the H1 peptide), the H1 peptide is selectively and reversibly bound to the FR3 or FR4 region of the light chain (corresponding to the L2 peptide), and the H2 peptide is selectively and reversibly bound to the FR2 region of the light chain (corresponding to the L1 peptide).

Referring to FIG. 4B, as a result of analyzing the hydrogen bond between H1-L2 peptides and the hydrogen bond between H2-L1 peptides through PyMOL, in the case of between H1-L2 peptides, (1) a hydrogen bond with a length of 3.34 Å between glutamine (residue number 9 of the H1 peptide) and tyrosine (residue number 3 of the L2 peptide) and (2) a hydrogen bond with a length of 3.39 Å between a carbonyl group of a peptide bond from the L2 peptide and an electron deficient hydrogen of the peptide bond from the H1 peptide were found, and in the case of between H2-L1 peptides, four hydrogen bonds with a length of 2.49 Å to 3.93 Å were found (1) between amino acids (interaction I), (2) between peptide bonds (interactions II, III), and (3) between amino acids and peptide bonds (interaction IV). The number of hydrogen bonds indicates that the interaction between the H1-L2 peptides is stronger than that between the H2-L1 peptides, whereas similar values of change in Gibbs free energy suggest similar binding affinities of peptide compounds to IgG.

FIG. 5 is graphs illustrating a fluorescence intensity measured at a bottom of a microplate after reacting lgGs from human, rabbit, goat, and mouse, which are isolated and immobilized on the microplate, respectively, and other proteins (human hepatitis B antigen, C-reactive protein, protein-A, BSA) with fluorescently labeled four types of peptide compounds (H1, H2, L1, L2) synthesized in Example 1.

Referring to FIG. 5, only IgGs from human, rabbit, goat, and mouse showed significantly high fluorescence intensities, and other antigenic proteins showed baseline level of fluorescence. These results show that the peptides of Example 1 are bound selectively to the antibody.

FIG. 6A is a schematic diagram illustrating states before and after treatment of the antibody to which the four types of peptides of Example 1 were bound, respectively, with papain, which is a protease, and FIG. 6B is graphs illustrating a fluorescence intensity measured at a bottom (B) of the microplate and in a solution (S) after the papain treatment. In the case of this experiment, after immobilization of the antibodies (rabbit anti-HRP antibodies) on the microplate, binding between the antibody and the switching peptide was induced by treatment with a fluorescently labeled switching peptide, the unbound switching peptide was removed, and then the Fab region was hydrolyzed from the immobilized antibodies by adding papain, which is a protease.

Referring to FIG. 6A, the fluorescence at the bottom (B) of the microplate is expected to decrease after the papain reaction because the fluorescently labeled switching peptides are bound to the Fab region of antibodies, and the fluorescence in the solution is expected to increase because the Fab region to which the switching peptide is bound is hydrolyzed and dissolved into the solution. This prediction is proven to be true from the graphs of FIG. 6B.

Referring to FIG. 6B, the fluorescence at the bottom (B) of the microplate decreased and the fluorescence in solution (S) increased, after papain treatment compared to before papain treatment in all four types of switching peptides (H1, H2, L1, L2) . Specifically, for the fluorescence intensity at the bottom of the microplate, it was evaluated that the H1 peptide decreased by 84.3%, the H2 peptide decreased by 58.7%, the L1 peptide decreased by 84.8%, and the L2 peptide decreased by 72.5%, respectively. Also, for the fluorescence intensity in the solution, it was evaluated that the H1 peptide increased by 2,184%, the H2 peptide increased by 2,356%, the L1 peptide increased by 1,032%, and the L2 peptide increased by 2,452%, respectively.

From the above results, it can be seen that the four types of switching peptides of Example 1 are bound to the Fab regions of the antibody.

Table 2 below shows the results of evaluating the binding properties of the four types of peptide compounds (H1, H2, L1, L2) of Example 1 to antibodies from different animals (rabbit, mouse, and goat). These results are fluorescence intensity values measured after binding fluorescently labeled switching peptides to antibodies immobilized on a microplate, removing unreacted switching peptides, and then reacting with target antigens (CRP for rabbit IgG, hCG for mouse IgG, HRP for rabbit IgG, HBsAg for goat IgG), respectively.

**[Table 2]**

| Pept ide | Antibody (r=rabbit , g-goat, m-mouse) | In solution (S) | | | Bottom (B) of microplate | | |
|---|---|---|---|---|---|---|---|
| | | Before antigen binding | After antigen binding | Change (%) | Before antigen binding | After antigen binding | Change (%) |
| H1 | Anti-HRP antibody ( r) | 2596 (±704) | 21965 (±7078) | +746 | 11840 (±290) | 1451 (±103) | -87.7 |
| | Anti-HBsA 9 antibody( g) | 2242(±91) | 11453(±829 ) | 1410 | 11225(±305 ) | 4618(±71) | -58.9 |
| | Anti-hCG antibody( m) | 1281(±63) | 3921(±120) | +206 | 5646(±20) | 1940(±74) | -65.6 |
| | Anti-CRP antibody( r) | 2242(±91) | 5659(±100) | 1152 | 4912(±1999 ) | 3595(±30) | -26.8 |
| H2 | Anti-HRP antibody ( r) | 2272(±668) | 18128(±145 6) | +698 | 122230(±30 9) | 1806(±290) | -85.2 |
| | Anti-HBsA g antibody ( g) | 1784(±113) | 9136(±835) | 1412 | 29367(±766 ) | 18773(±117 6) | -36.1 |
| | Anti-hCG antibody( m) | 1654(±54) | 8545(±1291 ) | +416 | 23320(±326 0) | 23320(±326 0) | -88.5 |
| | Anti-CRP antibody( r) | 2099(±120) | 16311(±165 ) | +677 | 22205(±732 ) | 22205(±732 ) | -39.5 |
| L1 | Anti-HRP antibody( r) | 2687 (±711) | 12280±(1314 1) | +357 | 15608(±638 ) | 1990 (±251) | -87.3 |
| | Anti-HBsA g antibody ( g) | 2779 (±293) | 20501(±228 5) | +638 | 11062(±462 6) | 5388 (±581) | -51.3 |
| | Anti-hCG antibody( m) | 1248 (±76) | 3580 (±416) | +187 | 4630 (±676) | 1777 (±44) | -61.6 |
| | Anti-CRP antibody( r) | 741(±197) | 6505(±61) | +778 | 6719(±132) | 3456(±178) | -48.6 |
| L2 | Anti-HRP antibody ( r) | 2315(±754) | 16135(±110 7) | +597 | 12584(±210 ) | 3208(±117) | -74.5 |
| | Anti-HBsA g antibody( g) | 2279(±635) | 15359(±405 0) | +574 | 28002(±316 ) | 9856(±1150 ) | -64.8 |
| | Anti-hCG antibody( m) | 1692(±126) | 9402(±656) | +456 | 33156(±498 0) | 3072(±184) | -90.7 |
| | Anti-CRP antibody ( r) | 3274(±353) | 32187(±138 35) | +883 | 20394(±54) | 11236(±72) | -44.9 |
| | | | | | | | |

Referring to Table 2, it was measured that the fluorescence intensity of each immobilized antibody (IgG) decreased at the bottom of the plate and increased in the solution after treatment with the corresponding antigen. Specifically, in the case of the anti-HRP antibodies from rabbits, for H1, L1, H2, and L2 peptides, the fluorescence intensity at the bottom of the plate decreased by 12.3%, 12.7%, 14.8%, and 25.5%, respectively, and the fluorescence intensity in the solution increased by 746%, 357%, 698%, and 597%, respectively. In the case of anti-HBsAg antibodies from goats, for H1, L1, H2, and L2 peptides, the fluorescence intensity at the bottom of the plate decreased by 41.1%, 48.7%, 63.9%, and 35.2%, respectively, and the fluorescence intensity in the solution increased by 410%, 638%, 412%, and 574%, respectively. These results show that the four types of peptide compounds synthesized in Example 1 are not only capable of binding to the immobilized antibody (IgG) regardless of the species of the source animal, but are also quantitatively released from the antibody when the antigen was bound to the binding pocket of the antibody due to the reversible binding of each of the four types of peptide compounds of Example 1 to the antibody.

FIG. 7A is a schematic diagram for explaining an immunoassay method using fluorescently labeled peptide compounds of Example 1, and FIGS. 7B and 7C are graphs illustrating the fluorescence intensities in a solution and at a bottom of the microplate depending on a concentration of the antigen measured by the immunoassay method illustrated in FIG. 7A. In this experiment, an anti-HRP antibody from a rabbit immobilized on a microplate was used as the binding antibody and HRP was used as the antigen.

Referring to FIGS. 7A to 7C, as a result of performing immunoassay while varying the concentration of antigen HRP from 0.3 to 100 µM, for the four types of switching peptides (H1, H2, L1, L2), an increase in the fluorescence intensity in the solution was observed depending on the concentration of the antigen (HRP).

Equilibrium dissociation constants of the H1, H2, L1, and L2 peptides were estimated to be 1.65, 3.11, 3.29, and 1.48 µM, and the difference in the dissociation constant for each switching peptide means that each switching peptide has a different detection area for each antigen. For the HRP antigen, the H1 peptide reacted most linearly in the HRP concentration region. These results show that the switching peptide was released from the immobilized antibody according to the bound amount of the antigen, and quantitative analysis of the antigen was possible based on the fluorescence intensity in the solution in one-step immunoassay using the switching peptide.

Meanwhile, FIG. 7C illustrated that the fluorescence intensity at the bottom of the microplate decreased depending on the concentration of the antigen in all four types of switching peptides. These results show that the switching peptide was quantitatively released from the antibody depending on the amount of the antigen, and quantitative analysis of the antigen was possible through a change in the fluorescence intensity in the solution as well as at the bottom of the microplate.

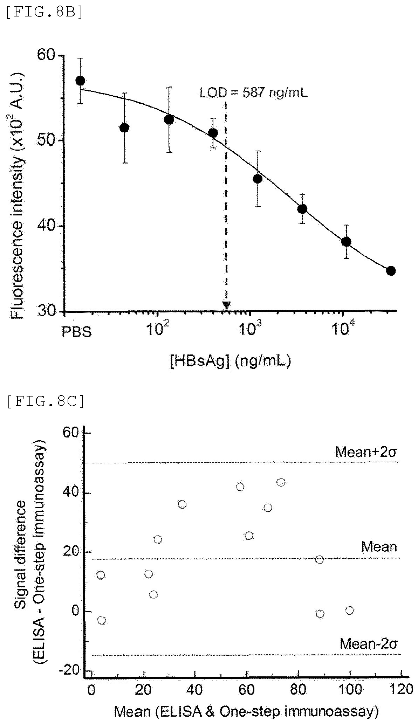

FIGS. 8A to 8D are experimental results for hepatitis B surface antigen (HBsAg) detected through immunoassay using the peptide compounds of Example 1. FIG. 8A is a graph illustrating the fluorescence intensity according to the concentration of the hepatitis B surface antigen, and FIG. 8B is a graph illustrating the fluorescence intensity of the hepatitis B surface antigen at the bottom of the microplate. In addition, FIG. 8C is a graph illustrating reliability in a Bland-Altman test of the one-step immunoassay, and FIG. 8D is a graph illustrating reliability in Passing-Bablok regression analysis.

After immobilizing the hepatitis B surface antigen (HBsAg) -antibody detected using the immunoassay of the peptide compounds of Example 1 on a microplate, L1 peptide was added, and 0.04 to 30 ug/ml of the hepatitis B surface antigen was treated. The L1 peptide was selected for the immunoassay, because the L1 peptide showed the highest selectivity among the four antigens of HRP, CRP, HBsAg, and hCG, as mentioned above.

Regarding the antibody binding properties of the switching peptides at pH 6.0 to 8.0, a net charge of H1 and L2 peptides decreased to 0.5 at pH 6.0 to 7.0 due to a histidine residue with a pKa value of 6.02. In addition, the net charge of the H2 and L2 peptides decreased to 0.5 at pH 7.0 to 8.0 due to a cysteine residue with a pKa value of 8.18. However, the L1 peptide showed almost the same fluorescence signal in a pH range required for immunoassay of hepatitis B surface antigen in human serum. Therefore, the immunoassay was performed by selecting an L1 peptide having specific dissociation from hepatitis B surface antigen-antibody binding and stability within a pH range of human serum immunoassay.

As illustrated in FIG. 8A, as a result of performing the immunoassay while varying the concentration of hepatitis B surface antigen (HBsAg) from 0.3 to 10⁵ ng/ml, an increase in the fluorescence intensity in the solution was observed depending on the concentration of the antigen (HBsAg) . In this case, the LOD is 56 ng/ml and the dynamic range is 136 ng/ml to 33 ug/ml. In addition, as illustrated in FIG. 8B, the fluorescence intensity at the bottom of the microplate decreased depending on the concentration of the antigen (HBsAg), and an LOD was observed to be 587 ng/ml. Accordingly, it can be confirmed that the antigen (HBsAg) is bound to the L1 peptide, and the degree of binding to the L1 peptide increases as the concentration of the antigen increases.

Then, for statistical comparison between ELISA and one-step immunoassay, a Bland-Altman test and Passing-Bablok regression analysis were performed using commercial software (MedCalc). As illustrated in FIG. 8C, the Bland-Altman test showed that the results of the one-step immunoassay and ELISA were the same, and were distributed within a confidence range of 95% (+1.96σ). As shown in FIG. 8D, as a result of Passing-Bablok regression analysis, the results of the two methods were distributed within a 95% confidence level, and a Spearman correlation coefficient (ρ) was calculated as 0.947 (P < 0.0001). Since the Spearman correlation coefficient (ρ) of 0.5 or more shows a high correlation between the two methods, the results of the two statistical analyzes indicate that the results of the ELISA and the immunoassay based on the switching peptide are similar. Therefore, it is shown that diagnosis of hepatitis B in humans based on surface antigen (HBsAg) detection is possible using the switching peptide according to the present disclosure.

FIGS. 9A to 9C are graphs illustrating a limit of detection and an immunoassay baseline in the one-step immunoassay using the switching peptide according to the present disclosure.

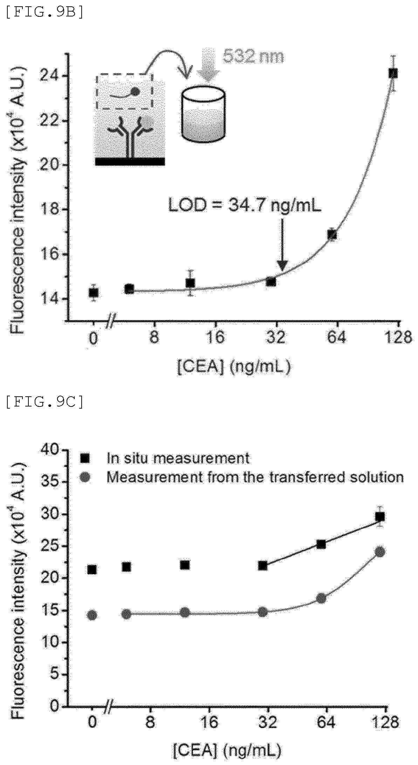

First, in the one-step immunoassay, carcinoembryonic antigen (CEA) was immobilized on the microplate and the L1 peptide was treated as an immobilized antibody. As illustrated in FIG. 9A, the fluorescence signal was measured in a microplate well state, and in this case, the limit of detection (LOD) was estimated to be about 33.6 ng/ml. In addition, when the fluorescence signal was measured from a transferred solution, the limit of detection (LOD) was estimated to be 34.7 ng/ml, as illustrated in FIG. 9B. Therefore, it is possible to quantitatively analyze carcinoembryonic antigen (CEA) through an immunoassay method using the switching peptide according to the present disclosure.

As illustrated in FIG. 9C, the baseline of the immunoassay was estimated to be 2.1 × 104 A.U. This seems to be due to the fact that there is no significant difference between the in situ measurement (FIG. 9A) and the transferred solution (FIG. 9B), and the fine difference is that the fluorescence signal decreases in proportion to the distance. Therefore, in the one-step immunoassay, quantitative analysis of the antigen using the switching peptide of the present disclosure indicates that it is possible even in a microplate well state without migration into a solution.

Although exemplary embodiments of the present disclosure have been disclosed hereinabove, it may be understood by those skilled in the art that the present disclosure may be variously modified and altered without departing from the scope and spirit of the present disclosure described in the following claims.

## Claims

1. A switching peptide comprising:
a peptide compound capable of reversibly binding to a binding antibody; and
a mass label bound to the peptide compound.

2. The switching peptide of claim 1, wherein the peptide compound is selectively and reversibly bound to a fragment antigen-binding (Fab) region of the binding antibody.

3. The switching peptide of claim 1, wherein the peptide compound has an amino acid sequence capable of specifically and reversibly binding to one or more of first to fourth framework regions (FR1, FR2, FR3, and FR4) of a light chain or heavy chain of the binding antibody.

4. The switching peptide of claim 3, wherein the peptide compound comprises one or more selected from the group consisting of a first peptide compound having a first amino acid sequence having homology to an amino acid sequence of the second light chain variable framework region (VL-FR2); a second peptide compound having a second amino acid sequence having homology to an amino acid sequence of the third or fourth light chain variable framework region (VL-FR3, VL-FR4); a third peptide compound having a third amino acid sequence having homology to an amino acid sequence of the second heavy chain variable framework region (VH-FR2); and a fourth peptide compound having a fourth amino acid sequence having homology to an amino acid sequence of the third or fourth heavy chain variable framework region (VH-FR3, VH-FR4).

5. The switching peptide of claim 4, wherein the peptide compound comprises 14 to 20 amino acids.

6. The switching peptide of claim 5, wherein the peptide compound has a molecular weight of 1,650 to 2,500 Da.

7. The switching peptide of claim 1, wherein the mass label comprises an amino acid compound.

8. A multiplex immunoassay method comprising:
a first step of immobilizing N different binding antibodies to which N different switching peptides are respectively bound, on a substrate;
a second step of treating the N binding antibodies with a detection sample solution containing one or more target antigens; and
a third step of performing quantitative analysis on the target antigens contained in the detection sample solution by identifying and quantitatively analyzing switching peptides released from the binding antibodies by mass spectrometry due to binding of the target antigens,
wherein each of the switching peptides comprises a peptide compound that reversibly bound to the binding antibodies and a mass label bound to the peptide compound, and
the mass labels of the switching peptides each comprise amino acid compounds having different molecular weights.

9. The multiplex immunoassay method of claim 8, wherein
the binding antibodies comprise first and second binding antibodies that are different from each other, and
the switching peptides comprise a first switching peptide comprising a first peptide compound that reversibly bound to the Fab region of the first binding antibody and a first mass label bound thereto and having a first molecular weight, and a second switching peptide comprising a second peptide compound that reversibly bound to the Fab region of the second binding antibody and a second mass label bound thereto and having a second molecular weight different from the first molecular weight,
wherein the first peptide compound and the second peptide compound are identical to each other.

10. The multiplex immunoassay method of claim 9, wherein
in the second step, the detection sample solution containing first and second target antigens that specifically react with the first and second binding antibodies is coated on the first and second binding antibodies to specifically react the first and second target antigens with the first and second binding antibodies, respectively, and
during the second step, the first switching peptide is quantitatively released from the first binding antibody depending on an amount of the first target antigen, and the second switching peptide is quantitatively released from the second binding antibody depending on an amount of the second target antigen.

11. The multiplex immunoassay method of claim 9, wherein during the third step, quantitative analysis of each of the first and second target antigens contained in the detection sample solution is performed by identifying and quantitatively analyzing the first and second switching peptides released from the first and second binding antibodies, respectively, by mass spectrometry.

12. The multiplex immunoassay method of claim 11, wherein mass spectrometry of the first and second switching peptides is performed by a method of laser desorption/ionization mass spectrometry or liquid chromatography mass spectrometry.

13. The multiplex immunoassay method of claim 11, wherein mass spectrometry of the first and second switching peptides is performed by a method of laser desorption/ionization mass spectrometry using an inorganic matrix,
wherein the inorganic matrix comprises semiconductor nanowires and porous metal nanoparticles bound to a surface of the semiconductor nanowires.
